(19) Europäisches Patentamt
European Patent Office
Office européen des brevets

(11) EP 3 110 395 B1

(12) **FASCICULE DE BREVET EUROPEEN**

(45) Date de publication et mention de la délivrance du brevet:
**30.01.2019 Bulletin 2019/05**

(21) Numéro de dépôt: **15709287.5**

(22) Date de dépôt: **20.02.2015**

(51) Int Cl.:
***A61K 8/49*** $^{(2006.01)}$ ***A61Q 5/00*** $^{(2006.01)}$
***A61K 8/81*** $^{(2006.01)}$ ***A61K 8/84*** $^{(2006.01)}$
***A61K 8/88*** $^{(2006.01)}$

(86) Numéro de dépôt international:
**PCT/FR2015/050412**

(87) Numéro de publication internationale:
**WO 2015/128566 (03.09.2015 Gazette 2015/35)**

# (54) PROCÉDÉ DE TRAITEMENT DES FIBRES KÉRATINIQUES AVEC UN POLYMÈRE AMINÉ ET UN ESTER ACTIVÉ

VERFAHREN ZUR BEHANDLUNG VON KERATINFASERN MIT EINEM AMINO-SUBSTITUIERTEN POLYMER UND EINEM AKTIVIERTEN ESTER

METHOD FOR TREATING KERATIN FIBRES WITH AN AMINO POLYMER AND AN ACTIVATED ESTER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.02.2014 FR 1451536**

(43) Date de publication de la demande:
**04.01.2017 Bulletin 2017/01**

(73) Titulaire: **L'Oréal**
**75008 Paris (FR)**

(72) Inventeurs:
- **JEGOU, Gwenaëlle**
  **F-91240 Saint Michel sur Orge (FR)**
- **BAGHDADLI, Nawel**
  **F-91300 Massy (FR)**

(74) Mandataire: **Rivière, François Armand**
**L'Oréal**
**Service DIPI**
**9 Rue Pierre Dreyfus**
**92110 Clichy (FR)**

(56) Documents cités:
**WO-A2-2008/156327 FR-A1- 2 910 276**
**FR-A2- 2 289 167 US-A- 4 713 236**

- **DATABASE WPI Week 201414 Thomson Scientific, London, GB; AN 2013-T28949 XP002731374, -& KR 2013 0114468 A (LG HOUSEHOLD & HEALTHCARE LTD) 18 octobre 2013 (2013-10-18)**
- **"Polylysine" In: Tara E. Gottschalck and Halyna P. Breslawec: "International Cosmetic Ingredient Dictionary and Handbook", 2012, Personal Care Products Council, Washington, D.C., XP002731375, vol. 2, pages 2540-2541, le document en entier**
- **"Vinylamine/vinylformamide copolymer" In: Tara E. Gottschalck and Halyna P. Breslawec: "International Cosmetic Ingredient Dictionary and Handbook", 2012, Personal Care Products Council, Washington, D.C., XP002731376, vol. 3, page 3462, le document en entier**
- **Janet Woodard: "Aziridine Chemistry - Applications for Cosmetics", J. Soc. Cosmet. Chem., 1 janvier 1972 (1972-01-01), pages 593-603, XP055147506, Extrait de l'Internet: URL:http://journal.scconline.org//pdf/cc1972/cc023n10/p00593-p00603.pdf [extrait le 2014-10-20]**
- **DATABASE WPI Week 198320 Thomson Scientific, London, GB; AN 1983-47418K XP002731377, -& JP S57 159706 A (TAMURA T) 1 octobre 1982 (1982-10-01)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 110 395 B1

## Description

La présente invention concerne un procédé de traitement des fibres kératiniques comprenant l'application sur les fibres d'un polymère aminé et d'un ester activé particuliers. Elle concerne également un kit permettant la mise en oeuvre d'un tel procédé.

Les cheveux sont généralement abîmés et fragilisés par l'action des traitements chimiques tels que les teintures, les décolorations, les permanentes, les défrisages, les lavages répétés.

Il est connu que certains traitements capillaires tels que les teintures, les décoloration, les permanentes, les défrisages peuvent agresser la fibre capillaire et notamment engendrer la perte de certains de ses constituants présents à l'état naturel, en particulier des acides gras tels que l'acide 18-méthyl eicosanoïque. Les cheveux se retrouvent endommagés et deviennent sensibilisés.

La fibre abimée présente un caractère électrostatique, rendant difficile la mise en forme correcte des cheveux, notamment lors du peignage ou du brossage. De plus, la fibre abimée présente un caractère plus hydrophile, la rendant très sensible à l'eau : la fibre a tendance à gonfler et à frisoter au contact de l'humidité ambiante, et n'assure pas dans ces conditions un bon maintien de la coiffure.

On sait que les qualités cosmétiques des cheveux peuvent être améliorées par application de diverses compositions à base d'actifs ou de polymères permettant de leur conférer diverses propriétés telles que brillance, facilité de démêlage, gonflant, souplesse, nervosité ou douceur. Pour obtenir une bonne efficacité, il convient bien entendu que ces actifs présentent vis-à-vis des fibres kératiniques, une certaine affinité.

Il est décrit dans le document WO2008/156327 une composition comprenant des lipides portant des groupes fonctionnels qui, après application sur les cheveux ou sur la peau, sont liés de façon covalente à la surface des matières kératiniques. Les groupes fonctionnels sont par exemple les groupes hydroxysuccinimidyl ester. Les lipides quant à eux sont des acides gras en $C_8$-$C_{28}$. Toutefois, l'effet d'hydrophobie obtenu en surface du cheveu s'estompe au cours des shampoings successifs et ne présente pas une rémanence au shampooing satisfaisante.

Par ailleurs, l'application de polymères traitants sur les cheveux peut nuire au maintien d'un bon toucher : les cheveux traités sont chargés, le toucher est rêche, peu lisse, crissant, peu cosmétique.

Il est également connu du document WO2008/156327 un procédé de traitement des cheveux par application d'un ester activé qui confère aux cheveux traités une hydrophobie de surface , pour la réparation et prévention des dommages, pour obtenir un effet brillant, soyeux, et un coating, et avec un effet de conditionnement semi-permanent, en particulier rémanent au shampooing.

Le document KR20130114468 décrit également un procédé de traitement des cheveux avec un ester activé similaire qui confère brillance, hydratation et résistance aux dommages.

Toutefois, ces traitements ne permettent pas d'obtenir des propriétés optimales d'hydrophobie et de rémanence. Il existe donc un besoin de disposer d'un procédé de traitement des cheveux permettant d'obtenir des propriétés d'hydrophobie et de rémanence améliorées.

La présente invention a pour but de proposer un procédé de traitement cosmétique des fibres kératiniques, notamment des cheveux, permettant de redonner à une fibre abimée, les propriétés physicochimiques de surface d'une fibre naturelle, et ce de façon durable dans le temps.

L'invention a ainsi pour objet un procédé cosmétique non thérapeutique de traitement des fibres kératiniques, comprenant dans l'ordre suivant :

une étape d'application sur les fibres kératiniques d'une première composition cosmétique comprenant un polymère aminé tel que défini ci-après,

suivi d'une étape d'application d'une deuxième composition cosmétique comprenant un ester activé tel que défini ci-après.

Le procédé de traitement est en particulier un procédé de soin des fibres kératiniques.

L'invention a également pour objet un kit comprenant :

une première composition cosmétique comprenant un polymère aminé tel que défini ci-après et une deuxième composition cosmétique comprenant un ester activé tel que défini ci-après, les premières et deuxième compositions étant conditionnées chacune dans un ensemble de conditionnement distinct.

L'ensemble de conditionnement des compositions est de façon connue tout packaging adapté pour stocker les compositions cosmétiques (flacons, tube, flacon spray, flacon aérosol notamment).

Un tel kit permet de mettre en oeuvre le procédé de traitement des matières kératiniques selon l'invention.

Il est connu des documents US4713236, FR2289167, FR2910276 des polymères à groupe amine primaire tels que polyéthylèneimines, polyallylimines pour améliorer les propriétés de conditionnement du cheveu.

Le procédé selon l'invention permet à la fibre abîmée de retrouver un état de surface hydrophobe proche de celui du cheveu naturel et ceci de manière durable l'effet d'hydrophobie étant rémanent après un ou plusieurs shampoings

effectués sur les fibres kératiniques traitées. L'apparence générale du cheveu se trouve améliorée, le cheveu traité est moins électrostatique, présente un moindre gonflement et moins de frisotis au contact de l'humidité ambiante, contribuant ainsi à une bonne mise en forme des cheveux, en particulier des cheveux fins. Les cheveux traités présentent également un bon toucher cosmétique, doux, non rêche. En particulier, comme le montre l'exemple 1, le procédé selon l'iinvention permet d'obtenir des propriétés optimales améliorées d'hydrophobie et de rémanence, tout en ayant un bon toucher. L'application seule de l'ester activé sur le cheveu ne permet pas d'atteindre la propriété otpimale d'hydrophobie et l'application seule du polymère aminé confère un toucher chargé, rêche donc peu cosmétique.

Le procédé selon l'invention met en oeuvre une première composition cosmétique comprenant un polymère aminé choisi parmi les polymères aminés à groupes amines primaires. Il s'agit de polymères issu de la polymérisation de monomères, le polymère comprenant au moins 2 groupes amines primaire $-NH_2$.

Le polymère aminé peut être notamment choisi parmi :

- les poly(alkylène ($C_2$-$C_5$) imines), et en particulier les polyéthylèneimines et les polypropylèneimines, notamment les poly(éthylène imine) linéaires ou ramifiés (par exemple celui vendu sous la référence 46,852-3 par la société Aldrich Chemical) ;
- la poly(allylamine) (par exemple celle vendue sous la référence 47,913-6 par la société Aldrich Chemical ou par la société Beckmann-Kenko) ;
- les polyvinylamines et leurs copolymères notamment avec des vinylamides; on peut notamment citer les copolymères vinylamine/vinylformamide (le taux de vinylamine pouvant aller de 30 à 90 % en poids du poids total du polymère) ; tels que ceux commercialisés sour la dénomination LUPAMIN® 9030 par la société BASF;
- les polyacides aminés présentant des groupes $NH_2$ comme la polylysine ; par exemple celle vendu par la société JNC Corporation ;
- l'amino dextrane, tel que celui vendu par la société CarboMer Inc ;
- les copolymères vinyl amine / alcool vinylique tel que ceux vendu par la société Chemwill ;
- les polymères d'acrylamidopropylamine ;
- les chitosanes, tels que ceux vendus sous les dénominations « ZENZIVO® Pro-tect » , « ZENZIVO Aqua » par la société Clariant.

De préférence, le polymère aminé est choisi parmi :

- les poly(éthylène imine) ;
- la poly(allylamine) ;
- la polylysine ;
- les copolymères vinylamine/vinylformamide ;
- les chitosanes.

Préférentiellement, le polymère aminé est choisi parmi la poly allylamine, , les copolymères poly(vinylamine/vinylformamide), les chitosanes.
Plus préférentiellement, le polymère aminé est une poly allylamine.

Avantageusement, le polymère aminé a un poids moléculaire moyen en poids allant de 200 à 1 000 000 g/mol, de préférence allant de 300 à 500 000 g/mol.

De préférence, le polymère aminé est hydrosoluble. On entend par polymère hydrosoluble un polymère ayant une solublité dans l'eau, à 25 °C, d'au moins 0,1 g/l.

Le polymère aminé peut être présent dans la première composition en une teneur allant de 0,001 à 20 % en poids, par rapport au poids total de la composition, de préférence allant de 0,001 à 10 % en poids, et préférentiellement allant de 0,001 à 5 % en poids, et plus préférentiellement allant de 0,1 à 5 % en poids.

La deuxième composition comprend un ester activé répondant à la formule (I) :

$$R\text{-}C(O)OA_1 \qquad (I)$$

dans laquelle R désigne un groupe alkyle en $C_5$-$C_{21}$, de préférence en $C_9$-$C_{17}$, préférentiellement en $C_{11}$-$C_{15}$ ;
$A_1$ désigne un groupe réactif choisi parmi :

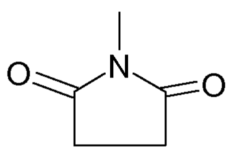 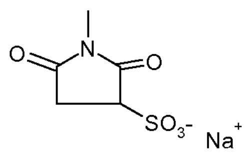 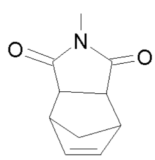 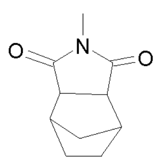

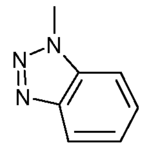

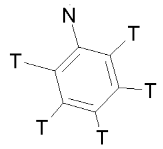

avec T= indépendamment H ou F ou Cl

Ces composés esters activés sont connus de la littérature.

De préférence , A1 désigne le groupe réactif

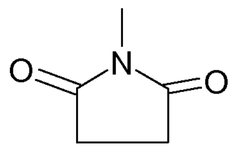

et R désigne un groupe alkyle en $C_9$-$C_{17}$, préférentiellement en $C_{11}$-$C_{15}$.

L'ester activé est de préférence choisi parmi : l'ester d'acide laurique et du N-hydroxy succinimide, l'ester d'acide palmitique et du N-hydroxysuccinimide. Avantageusement l'ester activé est l'ester d'acide palmitique et du N-hydroxysuccinimide.

L'ester activé peut être présent dans la deuxième composition en une teneur allant de 0,1 à 5 % en poids par rapport au poids total de la composition, de préférence allant de 0,5 à 4 % en poids, et préférentiellement allant de 1 à 4 % en poids.

Les compositions cosmétiques utilisées selon l'invention contiennent un milieu physiologiquement acceptable, c'est-à-dire compatible avec les matières kératiniques d'êtres humains tels que la peau (corps, visage, contour des yeux, cuir chevelu), les cheveux, les cils, les sourcils, les poils, les ongles, les lèvres.

Avantageusement, la première composition cosmétique utilisée selon l'invention comprend un milieu aqueux physiologiquement acceptable. Il peut par exemple être constitué par de l'eau ou par un mélange d'eau et d'au moins un solvant organique cosmétiquement acceptable. A titre de solvant organique, on peut par exemple citer les alcools inférieurs en $C_2$-$C_4$, tels que l'éthanol et l'isopropanol ; les polyols , notamment ceux ayant de 2 à 6 atomes de carbones comme la glycérine, le propylène glycol, le butylène glycol, le pentylène glycol, l'hexylène glycol, le dipropylène glycol, le diéthylène glycol ; les éthers de polyols comme le 2-butoxyéthanol, le monométhyléther de propylèneglycol, le monoéthyléther et le monométhyléther du diéthylèneglycol, les esters courts comme l'acétate d'éthyle, l'acétate de butyle; et leurs mélanges.

De préférence, la composition cosmétique comprend de 50 à 99,5% en poids d'eau par rapport au poids de la composition.

Avantageusement, la deuxième composition cosmétique utilisée selon l'invention comprend un milieu non aqueux physiologiquement acceptable. Il peut par exemple être constitué d'un ou plusieurs solvants organiques cosmétiquement acceptable, tels que ceux décrits précédemment, ou bien encore d'une ou plusieurs huiles usuelles en cosmétique.

Les compositions utilisées selon l'invention peuvent contenir en outre un ou plusieurs additifs cosmétiques choisi parmi les agents tensio-actifs non-ioniques, anioniques, cationiques et amphotères, les vitamines et pro-vitamines dont le panthénol, les filtres solaires, les charges, les matières colorantes, les agents nacrants, les agents opacifiants, les agents séquestrants, les polymères filmogènes, les agents plastifiants, les agents épaississants, les huiles, les agents antioxydants, les agents anti-mousse, les agents hydratants, les agents émollients, les agents de pénétration, les parfums et les agents conservateurs.

Les compositions utilisées selon l'invention peuvent se présenter sous toutes les formes galéniques classiquement utilisées pour une application capillaire et notamment sous forme de solutions aqueuses, solutions hydroalcooliques, d'émulsions huile-dans-eau (H/E) ou eau-dans-huile (E/H) ou multiple (triple : E/H/E ou H/E/H), de gels aqueux, de gels hydroalcooliques. Ces compositions sont préparées selon les méthodes usuelles. De préférence, la composition est sous forme de solution ou de gel aqueux ou hydroalcoolique.

Le procédé selon l'invention peut être mise en oeuvre sur des fibres kératiniques, notamment des cheveux, sèches ou humides. Préférentiellement, le procédé est mis en oeuvre sur fibres kératiniques, notamment des cheveux,

sèches.

Après application sur les fibres kératiniques de la composition cosmétique comprenant le polymère aminé et/ou l'ester activé, on peut laisser poser la composition appliquée pendant une durée allant de 1 à 60 minutes, de préférence allant de 2 à 50 minutes, préférentiellement allant de 5 à 30 minutes. Le temps de pose peut être effectué à une température allant de 15 °C à 45 °C, de préférence à la température ambiante (25 °C).

Les compositions cosmétiques décrites précédemment sont avantageusement appliquées sur les fibres kératiniques en une quantité allant de 0,1 à 10 grammes, de préférence de 0,2 à 5 grammes de composition par gramme de fibres kératiniques.

Après application des compositions cosmétique sur les fibres kératiniques, ces dernières peuvent être essorées pour retirer l'excès de composition ou bien lavées à l'eau.

Après le traitement, les fibres kératiniques peuvent être éventuellement rincées à l'eau ou lavées avec un shampooing. Les fibres kératiniques sont ensuite éventuellement séchées au moyen d'un sèche-cheveux ou d'un casque ou à l'air libre.

Le procédé de traitement selon l'invention est de préférence mis en oeuvre sur des fibres kératiniques, notamment des cheveux, sensibilisées telles que des fibres colorées artificiellement (fibres kératiniques teintes suite à un procédé de coloration directe ou par un procédé de coloration d'oxydation), décolorées, défrisées ou permanentées.

Le procédé de traitement selon l'invention peut être mis en oeuvre avant, pendant et/ou après un procédé de traitement cosmétique additionnel des fibres kératiniques, tels qu'un procédé de mise en forme temporaire (mise en forme avec bigoudis, fer à friser, fer à lisser) ou un procédé de mise en forme durable (permanente, défrisage) des fibres kératiniques.

Le procédé de traitement peut être mis en oeuvre en pré-traitement d'un procédé de coloration, de défrisage et/ou d'un procédé de permanente afin de protéger cosmétiquement les fibres kératiniques de ces traitements. En d'autres termes, ce procédé est effectué pour préserver les propriétés cosmétiques des fibres kératiniques avant un procédé de traitement cosmétique tel que décrit précédemment.

Préférentiellement, le procédé de traitement est mis en oeuvre en post-traitement d'un procédé de décoloration, de coloration artificielle, de défrisage et/ou d'un procédé de permanente afin de réparer lesdites fibres.

Le procédé selon l'invention est de préférence mis en oeuvre sur des cheveux.

Les exemples suivants sont donnés à titre illustratif de la présente invention. Les quantités indiquées dans les exemples sont exprimées en pourcentage en poids.

**Exemple 1** :

On a utilisé des mèches de cheveux sensibilisés (décoloration SA 20 %).

**Compositions réalisées**

| **Compositions** | **A1** | **A2** | **B** |
|---|---|---|---|
| Polyallylamine * | 0,5 | 3 | |
| Ester d'acide palmitique et de N-hydroxysuccinimide | | | 3 |
| Eau | qsp 100 | qsp 100 | |
| Acétate de butyle/éthanol (50/50) | | | qsp 100 |
| * polyallylamine de chez Beckmann-Kenko | | | |

Les essais ont été effectués sur une mèche de cheveu.

On a appliqué la composition A1 ou A2 sur des mèches de cheveux humidifiées (1,5 g de composition par gramme de cheveu), puis les mèches ont été placées en étuve à 60 °C pendant 30 minutes, en retournant les mèches au bout de 15 minutes.

On a ensuite appliqué sur les mèches la composition B (1,5 g de composition par gramme de cheveu). Les mèches ont ensuite été placées en étuve à 40 °C pendant 30 minutes, en les retournant au bout de 15 minutes.

On a ensuite rincées les mèches à l'eau courante , on les a essorées puis séchées au casque pendant 15 minutes à 60°C.

Pour certaines mèches, on a effectué 5 shampooings selon le protocole suivant :

Les mèches traitées ont été lavées avec un shampoing DOP camomille, à raison de 0,4 g de shampooing par gramme de cheveux, à la température de 38 °C.

Humidifier la mèche pendant 5 secondes avec de l'eau. Appliquer le shampooing en malaxant la mèche de la racine à la pointe pendant 15 secondes. Rincer à l'eau pendant 10 secondes. Essorer. Renouveler les étapes d'application du shamppoing suivi du rinçage. Sécher les mèches pendant 10 minutes par gramme de cheveux à 60 °C au sèche cheveux.

Pour chaque mèche de cheveux traitées, on a mesuré la mouillabilité du cheveu (mesure notamment décrite dans l'ouvrage « The science of Hair Care »de C. Bouillon et J.Wilkinson - 2ème édition 2005 - Chapitre 12 Evaluation of product efficacy - page 407).

La mesure de la mouillabilité d'un cheveu consiste à immerger un morceau de cheveu dans un cristallisoir d'eau ultra-pure et de mesurer la force engendrée par le déplacement de la fibre capillaire lors de son immersion (force de mouillage) et lors de son retrait de l'eau. Cette force varie en fonction de l'affinité du cheveu pour le liquide et permet de rendre compte de l'état de surface de la fibre.

Nous utilisons un tensiomètre K14 de la société KRÜSS dans les conditions opératoires suivantes :

- Vitesse d'approche: 4 mm/min
- Vitesse de mesure : 2 mm/min
- Sensibilité de la balance: 10 $\mu$g
- Temps d'attente après immersion: 2 secondes
- Profondeur d'immersion: 2 mm

Les cheveux montés sur un portoir sont toujours orientés dans le même sens à savoir, pointe vers le bas, racine vers le haut.

Pour chaque traitement 20 échantillons sont mesurés dans les mêmes conditions. On laisse les cheveux régulés toute une nuit dans une boite à gant à 25°C et 45 % d'hygrométrie avant de les mesurer.

Traitement des données.

La force de mouillage (F en Newton) est déterminée selon la formule suivante :

$$F = L \times \sigma \times \cos\theta$$

L étant le périmètre du cheveu (en mètre)

$\theta$ : angle de contact

$\Sigma$ : tension superficelle de l'eau pure = 72,75 mN/m à la pression atmosphérique

La force de mouillage exprimée en mN est transformée en $\mu$g, puis normalisée par rapport au périmètre moyen des fibres.

A partir des 20 mesures pour chaque mèche, la valeur moyenne et son intervalle de confiance sont calculés. Plus la force de mouilage est faible, plus le cheveu traité est hydrophobe.

On a également évalué le toucher de la mèche traitée (aspect plus ou moins rêche au toucher).

On a obtenu les résultats suivants :

| **Mèche / traitement** | **Toucher** | **Force de mouillage ($\mu$g)** |
|---|---|---|
| Mèche Témoin non traitée | Toucher correct | 5.39 ± 0.36 |
| Mèche 1 traitée avec composition B | Toucher correct | 3.59 ± 0.53 |
| Mèche 2 traitée avec composition A1 | Toucher plus rêche | -2.30 ± 0.35 |
| Mèche 3 traitée avec composition A1 + 5 shampooings | Toucher rêche | -6.28 ± 0.51 |
| Mèche 4 traitée avec composition A1 + 10 shampooings | Toucher rêche | -5.96 ± 0.82 |
| Mèche 5 traitée avec composition A1 puis B | Toucher correct | -3.49 ± 0.65 |
| Mèche 6 traitée avec composition A1 puis B + 5 shampooings | Toucher correct | -5.03 ± 0.43 |
| Mèche 7 traitée avec composition A1 puis B + 10 shampooings | Toucher correct | -4.76 ± 0.38 |
| Mèche 8 traitée avec composition A2 | Toucher chargé, rêche | -2.61 ± 0.60 |
| Mèche 9 traitée avec composition A2 + 5 shampooings | Toucher chargé, rêche | -6.34 ± 1.30 |

(suite)

| Mèche / traitement | Toucher | Force de mouillage (μg) |
|---|---|---|
| Mèche 10 traitée avec composition A2 puis B | Toucher correct | -4.43 ± 0.57 |
| Mèche 11 traitée avec composition A2 puis B + 5 shampooings | Toucher correct | -4.72 ± 0.62 |

Les résultats obtenus montrent que les mèches n° 5, 6, 7 et 10,11 traitées avec le procédé selon l'invention ont les meilleures propriétés d'hydrophobicité en surface et de bon toucher.

**Exemple 2 :**

Sur une mèche de cheveux sensibilisés (type SA 20), on applique la composition de basecoat suivante (teneurs en pourcentage pondéral) :

| | | |
|---|---|---|
| Polyéthylène imine de poids moléculaire 423 g/mol (reférence 46,853-3 de chez Aldrich) | | 0,5 % |
| Eau | qsp | 100 % |

On laisse poser pendant 30 minutes à 60 °C

On essuie l'excès de produit puis applique la composition de topcoat suivante :

| | | |
|---|---|---|
| ester d'acide palmitique et de N-hydroxysuccinimide | | 3 % |
| acétate de butyle/éthanol (50/50 poids/poids) | qsp | 00 % |

On laisse poser pendant 30 minutes.

On rince les cheveux traités puis on effectue un shampooing (DOP camomille) et les cheveux sont séchés au sèche-cheveux.

Les cheveux traités après l'application des 2 compositions présentent une hydrophobie de surface améliorée rémanente au shampooing ainsi qu'un bon toucher.

**Exemple 3 :**

Sur une mèche de cheveux sensibilisés (type SA 20), on applique la composition de basecoat suivante (teneurs en pourcentage pondéral) :
Copolymère poly(vinylamine/vinylformamide)

| | | |
|---|---|---|
| (LUPAMIN® 9030 de chez BASF) | | 0,5 % |
| Eau | qsp | 100 % |

On laisse poser pendant 30 minutes à 60 °C

On essuie l'excès de produit puis applique la composition de topcoat suivante :

| | | |
|---|---|---|
| ester d'acide palmitique et de N-hydroxysuccinimide | | 3 % |
| acétate de butyle/éthanol (50/50 poids/poids) | qsp | 00 % |

On laisse poser pendant 30 minutes.

On rince les cheveux traités puis on effectue un shampooing (DOP camomille) et les cheveux sont séchés au sèche-cheveux.

Les cheveux traités après l'application des 2 compositions présentent une hydrophobie de surface améliorée rémanente au shampooing ainsi qu'un bon toucher.

**Exemple 4 :**

Sur une mèche de cheveux sensibilisés (type SA20), on a appliqué la composition de basecoat suivante (teneurs en pourcentage pondéral) :

| | | |
|---|---|---|
| Polylysine (Polylysine 25 % solution de chez JNC) (reférence 47,913-6 de chez Aldrich) | | 0,5 % MA |
| Eau | qsp | 100 % |

On laisse poser pendant 30 minutes à 60 °C

On essuie l'excès de produit puis applique la composition de topcoat suivante :

| | | |
|---|---|---|
| ester d'acide palmitique et de N-hydroxysuccinimide | | 3 % |
| acétate de butyle/éthanol (50/50 poids/poids) | qsp | 00 % |

On laisse poser pendant 30 minutes.

On rince les cheveux traités puis on effectue un shampooing (DOP camomille) et les cheveux sont séchés au sèche-cheveux.

Les cheveux traités après l'application des 2 compositions présentent une hydrophobie de surface améliorée rémanente au shampooing ainsi qu'un bon toucher.

## Revendications

1. Procédé cosmétique non thérapeutique de traitement des fibres kératiniques, comprenant dans l'ordre suivant :

   une étape d'application sur les fibres kératiniques d'une première composition cosmétique comprenant un polymère aminé à groupe amine primaire,
   puis une étape d'application d'une deuxième composition cosmétique comprenant un ester activé de formule (I) :

   $$R\text{-}C(O)OA_1 \qquad (I)$$

   dans laquelle R désigne un groupe alkyle en $C_5$-$C_{21}$,
   $A_1$ désigne un groupe réactif choisi parmi :

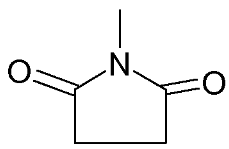
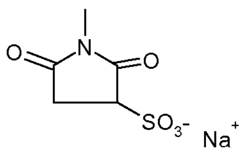
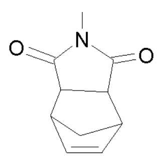
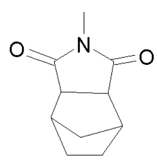

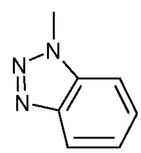

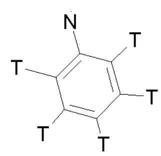

   avec T= indépendamment H ou F ou Cl

2. Procédé selon la revendication précédente, **caractérisé en ce que** le polymère aminé est choisi parmi :

- les poly(alkylène ($C_2$-$C_5$) imines) ;
- la poly(allylamine) ;
- les polyvinylamines, les les copolymères vinylamine/vinylformamide ;
- les polyacides aminés présentant des groupes $NH_2$ ;
- l'amino dextrane ;
- les copolymères vinyl amine / alcool vinylique ;
- les polymères à d'acrylamidopropylamine ;
- les chitosanes.

**3.** Procédé selon l'une quelconque des revendications précédentes, **caractérisé en ce que** le polymère aminé est choisi parmi :

- les poly(éthylène imine) ;
- la poly(allylamine) ;
- la polylysine ;
- les copolymères vinylamine/vinylformamide ;
- les chitosanes.

**4.** Procédé selon l'une quelconque des revendications précédentes, **caractérisé en ce que** le polymère aminé est une poly allylamine.

**5.** Procédé selon l'une quelconque des revendications précédentes, **caractérisé en ce que** le polymère aminé a un poids moléculaire moyen en poids allant de 200 à 1 000 000 g/mol, de préférence allant de 300 à 500 000 g/mol.

**6.** Procédé selon l'une quelconque des revendications précédentes, **caractérisé en ce que** le polymère aminé a une solublité dans l'eau, à 25 °C, d'au moins 0,1 g/l.

**7.** Procédé selon l'une quelconque des revendications précédentes, **caractérisé en ce que** le polymère aminé est présent dans la première composition en une teneur allant de 0,001 à 20 % en poids, par rapport au poids total de la composition, de préférence allant de 0,001 à 10 % en poids, et préférentiellement allant de 0,001 à 5 % en poids, et plus préférentiellement allant de 0,1 à 5 % en poids.

**8.** Procédé selon l'une quelconque des revendications précédentes, **caractérisé en ce que** pour l'ester activé de formule (I) R désigne un groupe alkyle en $C_9$-$C_{17}$, de préférence en $C_{11}$-$C_{15}$.

**9.** Procédé selon l'une quelconque des revendications précédentes, **caractérisé en ce que** pour l'ester activé de formule (I) A1 désigne le groupe réactif

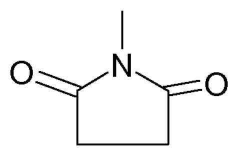

et R désigne un groupe alkyle en $C_9$-$C_{17}$, préférentiellement en $C_{11}$-$C_{15}$.

**10.** Procédé selon l'une quelconque des revendications précédentes, **caractérisé en ce que** l'ester activé de formule (I) est choisi parmi : l'ester d'acide laurique et du N-hydroxy succinimide, l'ester d'acide palmitique et du N-hydroxysuccinimide.

**11.** Procédé selon l'une quelconque des revendications précédentes, **caractérisé en ce que** l'ester activé de formule (I) est présent dans la deuxième composition en une teneur allant de 0,1 à 5 % en poids par rapport au poids total de la composition, de préférence allant de 0,5 à 4 % en poids, et préférentiellement allant de 1 à 4 % en poids.

**12.** Procédé selon l'une quelconque des revendications précédentes, **caractérisé en ce qu'**après application sur les fibres kératiniques de la première composition cosmétique comprenant le polymère aminé et/ou de la seconde composition comprenant l'ester activé, on peut laisser poser la composition appliquée pendant une durée allant de 1 à 60 minutes, de préférence à une température allant de 15 °C à 45 °C

**13.** Procédé selon l'une quelconque des revendications précédentes, **caractérisé en ce qu'**il est mis en oeuvre sur des cheveux.

**14.** Kit comprenant :
une première composition cosmétique comprenant un polymère aminé tel que défini selon l'une des revendications 1 à 7 et une deuxième composition cosmétique comprenant un ester activé tel que défini selon l'une des revendications 1 et 8 à 11 , les premières et deuxième compositions étant conditionnées chacune dans un ensemble de conditionnement distinct.

**Patentansprüche**

**1.** Kosmetisches, nicht therapeutisches Verfahren zur Behandlung von Keratinfasern, das in folgender Reihenfolge Folgendes umfasst:

einen Schritt zum Aufbringen einer ersten kosmetischen Zusammensetzung, die ein amino-substituiertes Polymer mit einer primären Aminogruppe umfasst, auf die Keratinfasern,
anschließend einen Schritt zum Aufbringen einer zweiten kosmetischen Zusammensetzung, die einen aktivierten Ester mit der Formel (I) umfasst:

$$R\text{-}C(O)OA_1 \qquad (I),$$

in der R eine Alkylgruppe mit $C_5$ bis $C_{21}$ bezeichnet,
$A_1$ eine reaktionsfähige Gruppe bezeichnet, ausgewählt aus:

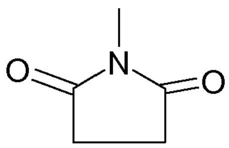
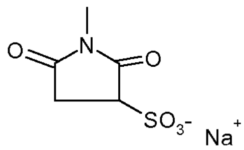
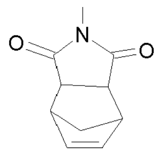
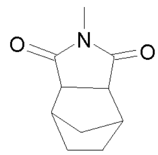

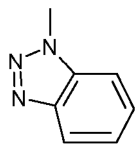

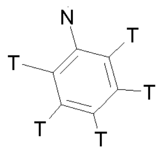

mit T = unabhängig H oder F oder Cl.

**2.** Verfahren nach dem vorhergehenden Anspruch, **dadurch gekennzeichnet, dass** das amino-substituierte Polymer ausgewählt wird aus:

- Poly (alkylen ($C_2$-$C_5$) iminen) ;
- Poly(allylamin);
- Polyvinylaminen, Vinylamin/Vinylformamid-Copolymeren;
- Polyaminosäuren mit $NH_2$-Gruppen;
- Aminodextran;
- Vinylamin/Vinylalkohol-Copolymeren;
- Acrylamidopropylamin-Polymeren;
- Chitosanen.

**3.** Verfahren nach einem der vorhergehenden Ansprüche, **dadurch gekennzeichnet, dass** das amino-substituierte

Polymer ausgewählt wird aus:

- Polyethyleniminen;
- Poly(allylamin);
- Polylysin;
- Vinylamin/Vinylformamid-Copolymeren;
- Chitosanen.

**4.** Verfahren nach einem der vorhergehenden Ansprüche, **dadurch gekennzeichnet, dass** das amino-substituierte Polymer ein Polyallylamin ist.

**5.** Verfahren nach einem der vorhergehenden Ansprüche, **dadurch gekennzeichnet, dass** das amino-substituierte Polymer eine gewichtsmittlere Molekülmasse von 200 bis 1.000.000 g/mol, vorzugsweise von 300 bis 500.000 g/mol aufweist.

**6.** Verfahren nach einem der vorhergehenden Ansprüche, **dadurch gekennzeichnet, dass** das amino-substituierte Polymer eine Löslichkeit in Wasser bei 25 °C von mindestens 0,1 g/l aufweist.

**7.** Verfahren nach einem der vorhergehenden Ansprüche, **dadurch gekennzeichnet, dass** das amino-substituierte Polymer in der ersten Zusammensetzung mit einem Gehalt von 0,001 bis 20 Gewichts-%, bezogen auf das Gesamtgewicht der Zusammensetzung, vorzugsweise von 0,001 bis 10 Gewichts-%, und vorzugsweise von 0,001 bis 5 Gewichts-%, und bevorzugter von 0,1 bis 5 Gewichts-% vorliegt.

**8.** Verfahren nach einem der vorhergehenden Ansprüche, **dadurch gekennzeichnet, dass** bei dem aktivierten Ester mit der Formel (I) R eine Alkylgruppe mit $C_9$ bis $C_{17}$, vorzugsweise mit $C_{11}$ bis $C_{15}$ bezeichnet.

**9.** Verfahren nach einem der vorhergehenden Ansprüche, **dadurch gekennzeichnet, dass** bei dem aktivierten Ester mit der Formel (I) A1 die reaktionsfähige Gruppe

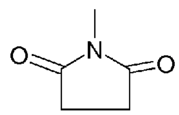

bezeichnet und R eine Alkylgruppe mit $C_9$ bis $C_{17}$ bezeichnet, vorzugsweise mit $C_{11}$ bis $C_{15}$.

**10.** Verfahren nach einem der vorhergehenden Ansprüche, **dadurch gekennzeichnet, dass** der aktivierte Ester mit der Formel (I) ausgewählt wird aus: dem Ester von Laurinsäure und von N-Hydroxysuccinimid, dem Ester von Palmitinsäure und von N-Hydroxysuccinimid.

**11.** Verfahren nach einem der vorhergehenden Ansprüche, **dadurch gekennzeichnet, dass** der aktivierte Ester mit der Formel (I) in der zweiten Zusammensetzung mit einem Gehalt von 0,1 bis 5 Gewichts-% bezogen auf das Gesamtgewicht der Zusammensetzung, vorzugsweise von 0,5 bis 4 Gewichts-% und vorzugsweise von 1 bis 4 Gewichts-% vorliegt.

**12.** Verfahren nach einem der vorhergehenden Ansprüche, **dadurch gekennzeichnet, dass** nach dem Aufbringen der ersten kosmetischen Zusammensetzung, die das amino-substituierte Polymer umfasst, und/oder der zweiten Zusammensetzung, die den aktivierten Ester umfasst, auf die Keratinfasern die aufgebrachte Zusammensetzung während einer Dauer von 1 bis 60 Minuten, vorzugsweise bei einer Temperatur von 15 °C bis 45 °C, ruhen gelassen werden kann.

**13.** Verfahren nach einem der vorhergehenden Ansprüche, **dadurch gekennzeichnet, dass** es auf Haaren durchgeführt wird.

**14.** Kit, umfassend:

eine erste kosmetische Zusammensetzung, die ein amino-substituiertes Polymer nach einem der Ansprüche 1 bis 7 umfasst, und eine zweite kosmetische Zusammensetzung, die ein aktiviertes Ester nach einem der

Ansprüche 1 und 8 bis 11 umfasst, wobei die erste und zweite Zusammensetzung jeweils in einer separaten Verpackungsanordnung verpackt sind.

## Claims

1. Non-therapeutic cosmetic process for treating keratin fibers, comprising, in the following order:

a step of applying, to the keratin fibers, a first cosmetic composition comprising an amino polymer comprising a primary amine group,
then a step of applying a second cosmetic composition comprising an activated ester of formula (I):

R-C(O)OA1 (I)

in which R denotes a $C_5$-$C_{21}$ alkyl group,
$A_1$ denotes a reactive group chosen from:

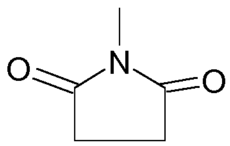

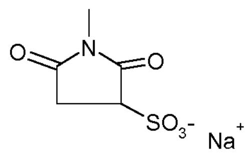

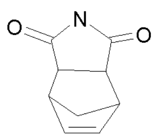

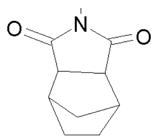

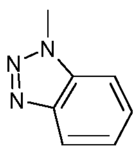

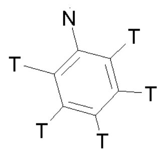

with T= independently H or F or Cl.

2. Process according to the preceding claim, **characterized in that** the amino polymer is chosen from:

- poly(($C_2$-$C_5$) alkyleneimine)s;
- poly(allylamine);
- polyvinylamines, vinylamine/vinylformamide copolymers;
- polyamino acids which have $NH_2$ groups;
- aminodextran;
- vinylamine/vinyl alcohol copolymers;
- acrylamidopropylamine polymers;
- chitosans.

3. Process according to either one of the preceding claims, **characterized in that** the amino polymer is chosen from:

- polyethyleneimines;
- poly(allylamine);

- polylysine;
- vinylamine/vinylformamide copolymers;
- chitosans.

**4.** Process according to any one of the preceding claims, **characterized in that** the amino polymer is a poly(allylamine) .

**5.** Process according to any one of the preceding claims, **characterized in that** the amino polymer has a weight-average molecular weight ranging from 200 to 1 000 000 g/mol, preferably ranging from 300 to 500 000 g/mol.

**6.** Process according to any one of the preceding claims, **characterized in that** the amino polymer has a solubility in water, at 25°C, of at least 0.1 g/l.

**7.** Process according to any one of the preceding claims, **characterized in that** the amino polymer is present in the first composition in a content ranging from 0.001% to 20% by weight, relative to the total weight of the composition, preferably ranging from 0.001% to 10% by weight, preferentially ranging from 0.001% to 5% by weight, and more preferentially ranging from 0.1% to 5% by weight.

**8.** Process according to any one of the preceding claims, **characterized in that**, for the activated ester of formula (I), R denotes a $C_9$-$C_{17}$, preferably $C_{11}$-$C_{15}$, alkyl group.

**9.** Process according to any one of the preceding claims, **characterized in that**, for the activated ester of formula (I), A1 denotes the reactive group

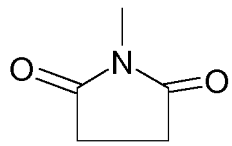

and R denotes a $C_9$-$C_{17}$, preferentially $C_{11}$-$C_{15}$, alkyl group.

**10.** Process according to any one of the preceding claims, **characterized in that** the activated ester of formula (I) is chosen from: the ester of lauric acid and of N-hydroxysuccinimide and the ester of palmitic acid and of N-hydroxysuccinimide.

**11.** Process according to any one of the preceding claims, **characterized in that** the activated ester of formula (I) is present in the second composition in a content ranging from 0.1% to 5% by weight, relative to the total weight of the composition, preferably ranging from 0.5% to 4% by weight, and preferentially ranging from 1% to 4% by weight.

**12.** Process according to any one of the preceding claims, **characterized in that**, after application to the keratin fibers of the first cosmetic composition comprising the amino polymer and/or of the second cosmetic composition comprising the activated ester, the applied composition may be left to stand on the fibers for a time ranging from 1 to 60 minutes, preferably at a temperature ranging from 15°C to 45°C.

**13.** Process according to any one of the preceding claims, **characterized in that** it is performed on hair.

**14.** Kit comprising:

a first cosmetic composition comprising an amino polymer as defined according to one of Claims 1 to 7 and a second cosmetic composition comprising an activated ester as defined according to one of Claims 1 and 8 to 11, the first and second compositions each being packaged in a separate packaging assembly.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2008156327 A
- KR 20130114468
- US 4713236 A
- FR 2289167
- FR 2910276


### Littérature non-brevet citée dans la description


- Evaluation of product efficacy. **C. BOUILLON ; J.WILKINSON.** The science of Hair Care. 2005, 407